# EUROPÄISCHE PATENTSCHRIFT

(11) **EP 2 563 283 B1**
(45) Veröffentlichungstag und Bekanntmachung des Hinweises auf die Patenterteilung: **12.08.2015**
(21) Anmeldenummer: 11726681.7
(22) Anmeldetag: 26.04.2011
(51) Int. Cl.: A61F 2/44

(54) **WIRBELSÄULENIMPLANTAT UND WERKZEUG HIERFÜR**
VERTEBRAL COLUMN IMPLANT AND TOOL FOR SAID IMPLANT
IMPLANT POUR COLONNE VERTÉBRALE ET OUTIL À CET EFFET

(30) Priorität: 26.04.2010 DE 102010018379
(43) Veröffentlichungstag der Anmeldung: 06.03.2013
(73) Patentinhaber: Metz-Stavenhagen, Peter, 34537 Bad Wildungen (DE)
(72) Erfinder: Metz-Stavenhagen, Peter, 34537 Bad Wildungen (DE)
(74) Vertreter: Walther, Walther & Hinz GbR
(86) Internationale Anmeldenummer: PCT/DE2011/000439
(87) Internationale Veröffentlichungsnummer: WO 2011/134457

(56) Entgegenhaltungen:
- EP-A1- 1 219 266
- EP-A1- 2 055 270
- WO-A1-2009/151734
- WO-A2-2008/121312
- US-A1- 2002 082 696

## Beschreibung

Die vorliegende Erfindung betrifft ein Wirbelsäulenimplantat gemäß dem Oberbegriff des Anspruches 1, sowie ein Werkzeug hierfür gemäß dem Oberbegriff des Anspruches 9 und ein Verfahren zum Distrahieren des Wirbelsäulenimplantates gemäß dem Oberbegriff des Anspruches 14.

Aus der US 7,029,498 B2 ist ein aus zwei U-förmigen Teilen zusammengesetztes, distrahierbares Wirbelsäulenimplantat bekannt, bei dem die beiden Teile teleskopartig gegeneinander axial verschiebbar gehalten sind. An den freien Stegen des U-förmigen Außenteiles ist eine Transportaufnahme ausgebildet, in die eine Faßzange einsetzbar ist. Mit dieser Faßzange kann der behandelnde Arzt das Wirbelsäulenimplantat ergreifen und zum gewünschten Ort transportieren.

Nachdem das Wirbelsäulenimplantat platziert ist wird die Faßzange wieder entfernt. Zum Distrahieren des Wirbelsäulenimplantates auf die gewünschte Größe wird als nächstes ein länglicher Führungsstab an der Öffnung des U-förmigen Teiles vorbei ins Innere des Wirbelsäulenimplantates geführt und in ein am Außenteil vorhandenes Gewinde eingeschraubt, bevor ein hohles Verzahnungsinstrument über den Führungsstab geschoben wird. Dabei wird das Verzahnungsinstrument soweit in das Wirbelsäulenimplantat eingeschoben, bis an dem Verzahnungsinstrument vorhandene Außenzähne in entsprechend ausgebildete Zähne am Innenteil des Wirbelsäulenimplantates eingreifen. Dreht man nun das Verzahnungsinstrument um seine Längsachse, so wird das Innenteil des Wirbelsäulenimplantates gegenüber dem Außenteil verschoben.

Dieser ganze Vorgang ist sehr schwierig und erfordert ein hohes Maß an Fingerfertigkeit des operierenden Arztes. Weil das Verzahnungsinstrument nur sehr lose auf dem Führungsstab sitzt kann es während dem Distrahieren vorkommen, dass es unbeabsichtigterweise aus dem Eingriff mit den Zähnen herausrutscht, so dass es neu eingeführt werden muss.

Aus der EP 2 055 270 A1 ist ein Handhabungswerkzeug für ein medizinisches Implantat, sowie das dazugehörige Implantat bekannt. Das medizinische Implantat setzt sich aus zwei im Wesentlichen U-förmigen Bauteilen zusammen, die derart ineinander geschoben werden können, dass die jeweiligen freien Stege versetzt angeordnet sind. Durch eine Öffnung eines ersten Steges kann dann das Handhabungswerkzeug bis in das dahinterliegende Bauteil reichen und greift dort in einen losen auf dem anderen Bauteil aufsitzenden Ring ein. Durch Verdrehen des Werkzeuges wird der Ring gedreht und die beiden Bauteile schrauben sich auseinander. Die Handhabung dieses Werkzeuges ist sehr mühsam und dabei ist auch eine Einhandbedienung nicht möglich. Außerdem kann das Implantat licht derart am Werkzeug fixiert werden, dass ein Tränsport möglich ist.

Davon ausgehend liegt der vorliegenden Erfindung die Aufgabe zu Grunde, ein Wirbelsäulenimplantat und ein Werkzeug hierfür der eingangs genannten Art zu schaffen, so dass das Wirbelsäulenimplantat in einfacher Weise und präzise implantiert und distrahiert werden kann.

Als technische Lösung dieser Aufgabe wird erfindungsgemäß ein Wirbelsäulenimplantat der eingangs genannten Art mit den Merkmalen des Anspruches 1 und ein Werkzeug mit den Merkmalen des Anspruches 9, sowie ein Verfahren zum Distrahieren des Wirbelsäulenimplantates gemäß den Merkmalen des Anspruches 14 vorgeschlagen. Vorteilhafte Weiterbildungen dieses Wirbelsäulenimplantates, dieses Werkzeuges und dieses Verfahrens sind den jeweiligen Unteransprüchen zu entnehmen.

Ein nach dieser technischen Lehre ausgebildetes Wirbelsäulenimplantat hat den Vorteil, dass mit nur einem Werkzeug sowohl der Transport, als auch die Distraktion durchgeführt werden kann. Dies erspart insbesondere das mühsame Einführen des zweiten Instrumentes nach dem Platzieren des Implantates.

Ein weiterer Vorteil des erfindungsgemäßen Wirbelsäulenimplantates besteht darin, dass das Wirbelsäulenimplantat mit einem einzigen Werkzeug zuverlässig gehalten, transportiert und implantiert werden kann und dass das Wirbelsäulenimplantat mit dem gleichen Werkzeug auch positioniert und distrahiert werden kann, vor allem aber, dass das Werkzeug mit einer einzigen Hand bedient werden kann, so dass der operierende Arzt die andere Hand für andere Tätigkeiten zur Verfügung hat.

Noch ein weiterer Vorteil besteht darin, dass am Innen- und am Außenkorpus Führungsmittel vorgesehen sind, die ein Verdrehen des Innenkorpus gegenüber dem Außenkorpus verhindern und gleichzeitig eine axiale Führung gewährleisten. Dies hat den Vorteil, dass beim Distrahieren etwaig auftretende Umfangskräfte nicht zum Verdrehen des Innenkorpus führen. Es hat sich als vorteilhaft erwiesen, diese Führungsmittel nach dem Nut-Feder-Prinzip auszugestalten. Dabei ist beispielsweise im Innenkorpus eine koaxial angeordnete Nut ausgebildet, in die einen korrespondierend hierzu ausgebildeten Vorsprung an einer Innenwandung des Außenkorpus eingreift. Durch diese Nut-Feder Konstruktion erfolgt die Führung des Innenkorpus über eine gewisse Länge des Wirbelsäulenimplantates, so dass ein Verkanten zuverlässig verhindert wird.

In einer bevorzugten Weiterbildung sind am Werkzeug und am Implantat Mittel zum Arretieren ausgebildet, mit der das Werkzeug am Implantat arretiert werden kann. Mit einem derart am Implantat arretierten Werkzeug ist es auch möglich, das Implantat mit nur einer Hand im Körper zu bewegen, um es exakt zu positionieren. Dabei spielt es keine Rolle, ob das Werkzeug am Innen- oder am Außenkorpus arretiert wird. In einer bevorzugten Ausführungsform erfolgt das Arretieren über einen Drehverschluss, wobei in der Transportaufnahme ein Innengewinde ausgebildet ist, während am Transportstab des Werkzeuges ein entsprechendes Außengewinde vorgesehen ist. Mit einem derartigen Drehverschluss kann das Werkzeug schnell und in einfacher Weise an dem Implantat arretiert werden. Hierdurch kann das Werkzeug auch zum Halten des Implantates eingesetzt werden. Bei einem derart mit dem Implantat verbundenen Werkzeug kann vom behandelnden Arzt das Implantat mit nur einer Hand transportiert oder neu positioniert werden.

Alternativ zum Drehverschluss können die Mittel zur Arretierung auch als Bajonettverschluss ausgebildet sein, bei dem in der Transportaufnahme eine Bajonettaufnahme ausgebildet ist, während am Werkzeug korrespondierende Bajonettstege vorgesehen sind. Mit einem derartigen Bajonettverschluss kann das Werkzeug schnell und in einfacher Weise am Implantat angebracht werden.

Eine weitere Alternative besteht darin, die Mittel zum Arretieren als Zahnverschluss auszubilden, wobei am Werkzeug ein Außenzahnkranz ausgebildet ist, dessen Zähne in korrespondierende Zähne des Innenzahnkranzes der Transportaufnahme eingreifen, um einen Formschluss herzustellen. Noch ein weiterer Vorteil besteht darin, dass es Aufgrund des formschlüssigen Eingriffes des Werkzeuges in die Wandungszähne auch möglich, den Innenkorpus, bzw. den Außenkorpus, mit geringem Kraftaufwand zu distrahieren, selbst wenn dieser bereits implantiert ist und sogar, wenn dieser unter Belastung steht. Weitere Vorteile des erfindungsgemäßen Wirbelsäulenimplantates, des erfindungsgemäßen Werkzeuges und des erfindungsgemäßen Verfahrens ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine perspektivische Darstellung einer ersten Ausführungsform eines erfindungsgemäßen Wirbelsäulenimplantates und eines erfindungsgemäßen Werkzeuges;
- Fig. 2: eine geschnitten dargestellte Draufsicht auf das Wirbelsäulenimplantat und das Werkzeug gemäß Fig. 1, geschnitten entlang Linie II - II in Fig. 1;
- Fig. 3: eine geschnitten dargestellte Seitenansicht des Wirbelsäulenimplantates gemäß Fig. 1, geschnitten entlang Linie III - III in Fig. 1;
- Fig. 4: eine geschnitten dargestellte Draufsicht auf eine zweite Ausführungsform eines erfindungsgemäßen Wirbelsäulenimplantates und eines erfindungsgemäßen Werkzeuges;
- Fig. 5: eine geschnitten dargestellte Draufsicht auf das Wirbelsäulenimplantat gemäß Fig. 4;
- Fig. 6: eine perspektivische Darstellung einer dritten Ausführungsform eines erfindungsgemäßen Wirbelsäulenimplantates und eines erfindungsgemäßen Werkzeuges;
- Fig. 7: eine geschnitten dargestellte Draufsicht auf das Wirbelsäulenimplantat und das Werkzeug gemäß Fig. 6, geschnitten entlang Linie VII - VII in Fig. 6;
- Fig. 8: eine geschnitten dargestellte Seitenansicht des Wirbelsäulen-implantates gemäß Fig. 6, geschnitten entlang Linie VIII - VIII in Fig. 6;

- Fig. 9: eine geschnitten dargestellte Draufsicht auf eine vierte Ausführungsform eines erfindungsgemäßen Wirbelsäulenimplantates und eines erfindungsgemäßen Werkzeuges;
- Fig. 10: eine geschnitten dargestellte Draufsicht auf das Wirbelsäulenimplantat gemäß Fig. 9.
- Fig. 11: eine perspektivische Darstellung einer fünften Ausführungsform eines erfindungsgemäßen Wirbelsäulenimplantates und des erfindungsgemäßen Werkzeuges;
- Fig. 12: eine geschnitten dargestellte Draufsicht auf das Wirbelsäulenimplantat und das Werkzeug gemäß Fig. 11, geschnitten entlang Linie XII - XII in Fig. 11;
- Fig. 13: eine geschnitten dargestellte Seitenansicht des Wirbelsäulen-implantates gemäß Fig. 11, geschnitten entlang Linie XIII - XIII in Fig. 11.

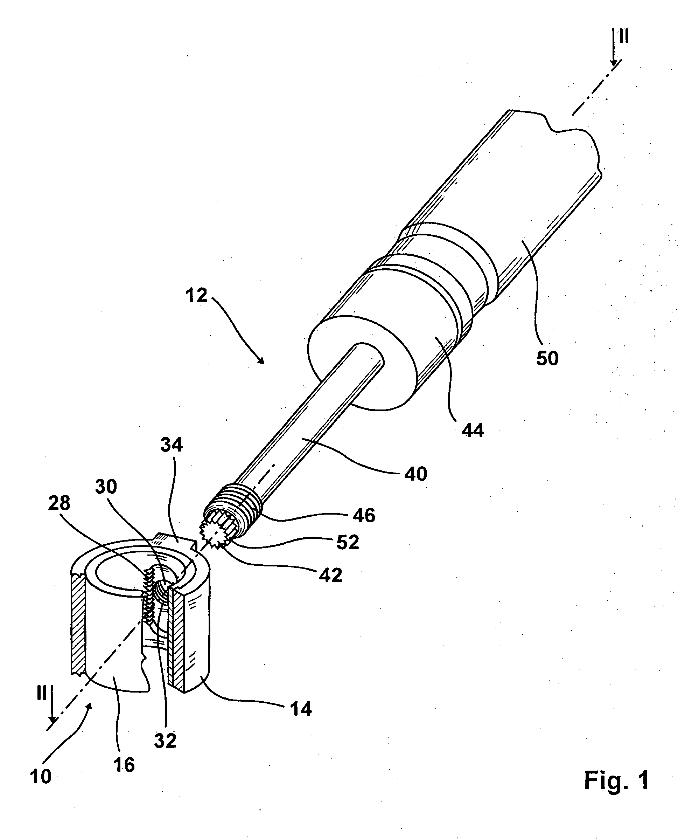

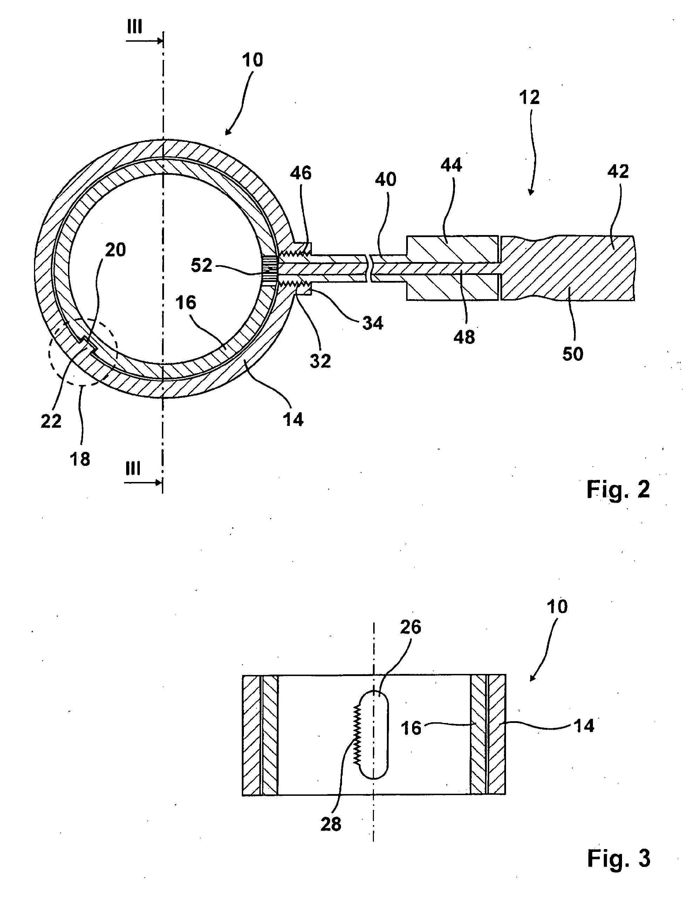

In den Fig. 1 bis 3 ist eine erste Ausführungsform eines erfindungsgemäßen Wirbelsäulenimplantates 10 und eine Ausführungsform eines erfindungsgemäßen Werkzeuges 12 dargestellt.

Das Wirbelsäulenimplantat umfasst einen Außenkorpus 14 und einen axial verschieblich darin gehaltenen Innenkorpus 16, die beide in der hier dargestellten Ausführungsform zylindrisch ausgeführt sind. An den beiden Korpi 14, 16 sind Führungsmittel 18 ausgebildet, die die axiale Bewegung der beiden Korpi 14, 16 führt und ein Verdrehen der Korpi 14, 16 gegeneinander sperrt. Das Führungsmittel 18 umfasst eine an einer Außenseite des Innenkorpus 16 eingelassene, koaxial ausgerichtete Nut 20 und einen korrespondierend zur Nut 20 an einer Innenseite des Außenkorpus 14 ausgebildeten Vorsprung 22.

Im Innenkorpus 16 ist eine als koaxial ausgerichtetes Langloch ausgebildete Hebelaufnahme 26 ausgebildet, an dessen rechter oder linker vertikalen Flanke eine Anzahl von Wandungszähnen 28 ausgebildet sind.

Im Außenkorpus 14 ist eine als Öffnung ausgebildete Transportaufnahme 30 vorgesehen, in der ein Innengewinde 32 eingelassen ist. Im Bereich dieser Transportaufnahme 30 ist der Außenkorpus 14 verstärkt ausgeführt, so dass sich hier ein flanschähnlicher Ansatz 34 ausbildet.

Die Nut 20, der Vorsprung 22, der Innenkorpus 16 und der Außenkorpus 14 sind so angeordnet, dass die als Langloch ausgebildete Hebelaufnahme 26 mit der als Öffnung ausgebildeten Transportaufnahme 30 fluchtet, so dass ein Werkzeug 12 durch die Transportaufnahme 30 hindurch bis in die Hebelaufnahme 26 gesteckt werden kann.

Das Werkzeug 12 setzt sich zusammen aus einer Instrumentenaufnahme mit einem Transportstab 40 und einem Hebeldreher 42, sowie einem Griff. der Griff ist zweiteilig ausgebildet und setzt sich aus einem Transportgriff 44 und einem Hebelgriff 50 zusammen.

Der Transportstab 40 umfasst einen am proximalen Ende ausgebildeten Transportgriff 44 und ein am distalen Ende angebrachtes Außengewinde 46. Der Hebeldreher 42 umfasst einen länglichen Schaft 48, an dessen proximalem Ende ein Hebelgriff 50 und an dessen distalem Ende eine Anzahl Umfangszähne 52 angebracht sind.

Der Transportstab 40 ist innen hohl ausgeführt und nimmt in diesem Hohlraum den Schaft 48 des Hebeldrehers 42 auf. Dabei ist der Schaft 48 so gewählt, dass die am distalen Ende des Schaftes 48 radial abstehenden Umfangszähne 52 einerseits und der Hebelgriff 50 andererseits den Hebeldreher 42 passgenau und formschlüssig am Transportstab 40 halten. Durch diesen zweiteiligen Aufbau des Werkzeuges 12 ist es möglich, den Transportstab 40 unabhängig vom Hebeldreher 42 zu bewegen.

In der in den Fig. 1 bis 3 dargestellten Ausführungsform sind die radial abstehenden Umfangszähne 52 über den gesamten Umfang des Schaftes 48 verteilt, so dass ein kompletter Kranz von Umfangszähnen 52 entsteht. Bei der in den Fig. 1 bis 3 dargestellten ersten Ausführungsform kann das Werkzeug 12 mittels eines Drehverschlusses am Implantat gehalten werden. Dieser Drehverschluss umfasst das am Transportstab 40 des Werkzeug 12 angebrachte Außengewinde 46 sowie das in der Öffnung der Transportaufnahme 30 des Außenkorpus 14 angeordnete Innengewinde 32, wobei das Außengewinde 46 und das Innengewinde 32 aufeinander abgestimmt sind. Nachfolgend wird kurz beschrieben, wie das Werkzeug 12 am Wirbelsäulenimplantat 10 verwendet werden kann:

Während eines chirurgischen Eingriffes bestimmt der behandelnde Arzt die Größe des einzusetzenden Wirbelsäulenimplantates 10. Dann kann, zum Beispiel von einem Helfer, das Werkzeug 12 mit seinem Transportstab 40 am Wirbelsäulenimplantat 10 angebracht werden. Dies geschieht durch Einschrauben des am distalen Ende des Transportstabs 40 befindlichen Außengewindes 46 in das Innengewinde 32 am Außenkorpus 14. Hierdurch entsteht eine formschlüssige und sehr stabile Verbindung zwischen dem Werkzeug 12 und dem Wirbelsäulenimplantat 10.

Zu gegebener Zeit kann der behandelnde Arzt nun das Werkzeug 12 am Griff, wahlweise am Transportgriff 44 und/oder am Hebelgriff 50 ergreifen und hält somit nicht nur das Werkzeug 12, sondern auch das daran angeschraubte Wirbelsäulenimplantat 10 fest in der Hand. Hierzu benötigt der behandelnde Arzt nur eine einzige Hand und kann mit der anderen Hand andere Tätigkeiten ausführen.

Das über das Werkzeug 12 ergriffene Wirbelsäulenimplantat 10 kann der behandelnde Arzt nun zu der gewünschten Stelle im Körper des Patienten bringen und dort wunschgemäß platzieren. Durch die formschlüssige Verbindung zwischen dem Werkzeug 12 und dem Wirbelsäulenimplantat 10 ist es möglich, dass der behandelnde Arzt mit einer einzigen Hand Kräfte auf das Wirbelsäulenimplantat 10 ausübt, um dies auch unter schwierigen operativen Bedingungen an der gewünschten Stelle und in der gewünschten Form zu platzieren.

Ist das Wirbelsäulenimplantat 10 implantiert, so kann eine Distraktion des Wirbelsäulenimplantates 10 dadurch erfolgen, dass der behandelnde Arzt den Hebelgriff 50 des Hebeldrehers 42 ergreift und in der gewünschten Richtung verdreht. Dabei greifen die am distalen Ende des Hebeldrehers 42 angebrachten Umfangszähne 52 in die Wandungszähne 28 am Innen-korpus 16 ein und verschieben diesen axial. Ein Ausweichen des Innen-korpus 16 gegenüber dem Außenkorpus 14 wird durch das Führungsmittel 18, insbesondere durch die Nut 20 und den Vorsprung 22 verhindert, so dass eine exakte axiale Verschiebung des Innenkorpus 16 erfolgt.

Durch die Hebelwirkung des Hebeldrehers 42 kann der Innenkorpus 16 auch dann entsprechend distrahiert werden, wenn auf dem Wirbelsäulen-implantat 10 bereits eine Belastung anliegt. Ein Verkanten des Wirbelsäulenimplantates 10 wird unter anderem durch das Führungsmittel 18 verhindert.

Nachdem das Wirbelsäulenimplantat 10 in angemessener Weise distrahiert ist, kann dieses durch Einsetzen entsprechender Sicherungsschrauben in der gewünschten Position fixiert werden. Anschließend wird das Werkzeug 12 durch Rückdrehen des Transportstabs 40 aus dem Außenkorpus 14 des Wirbelsäulenimplantates 10 herausgedreht und kann vollständig entnommen werden.

Sollte eine Korrektur des Wirbelsäulenimplantates 10 erforderlich sein, so kann dies in analoger Weise mit dem Werkzeug 12 durchgeführt werden.

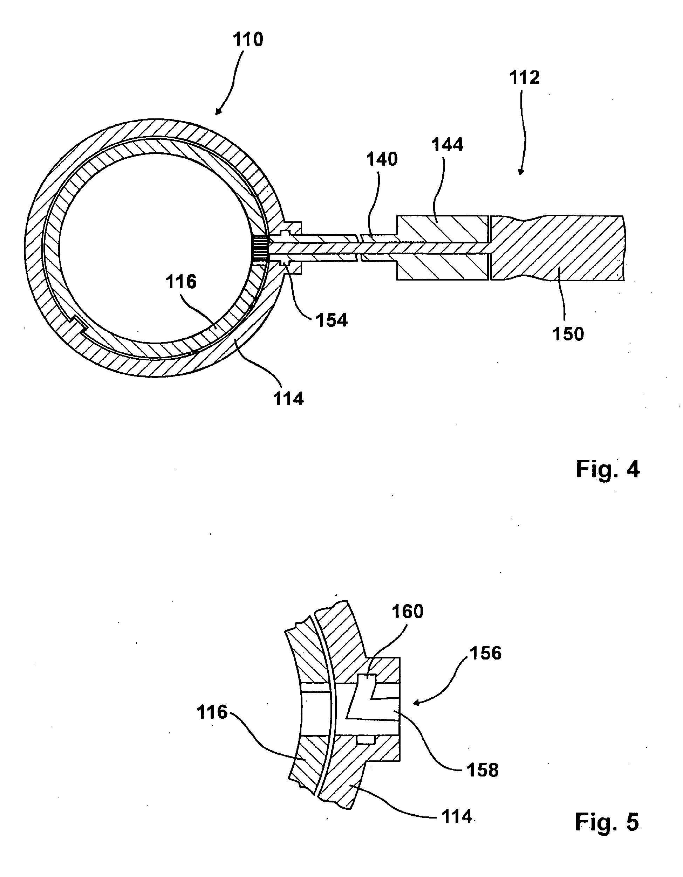

Die in den Fig. 4 und 5 dargestellte zweite Ausführungsform unterscheidet sich von der in den Fig. 1 bis 3 dargestellten Ausführungsform lediglich dadurch, dass die Mittel zum Arretieren nicht als Drehverschluss, sondern als Bajonettverschluss ausgeführt ist. Zu diesem Bajonettverschluss gehören zwei radial vom distalen Ende des Transportstabes 140 des Werkzeuges 112 abstehende Bajonettstege 154 und entsprechende, in der Transportaufnahme 130 des Außenkorpus 114 des Wirbelsäulenimplanta-tes 110 eingelassene Bajonettaufnahmen 156. Zur Anbringung des Werkzeuges 112 am Wirbelsäulenimplantat 110 wird das Werkzeug 112 mit einer Hand am Griff ergriffen und das freie Ende des Transportstabes 140 wird mit den Bajonettstegen 154 in die Bajonettaufnahmen 156 in der Transportaufnahme 130 des Außenkorpus 114 eingeführt. Anschließend wird der Transportstab 140 um seine Längsachse verdreht, so dass die Bajonettstege 154 tief in die Bajonettaufnahmen 156 eingeführt werden.

Wie insbesondere Fig. 5 zu entnehmen ist, umfasst die im Außenkorpus 114 ausgebildete Bajonettaufnahme 156 einen trichterförmig zulaufenden Eingangskanal 158, um das Einfädeln der Bajonettstege 154 zu erleichtern und einen Arretierkanal 160, wobei der Arretierkanal 160 in einem spitzen Winkel zum Eingangskanal 158 angeordnet ist. Die Anordnung in einem spitzen Winkel von vorzugsweise 80° hat den Vorteil, dass der auf den Arretierstab 140 drückende Innenkorpus 116 die Bajonettstege 154 in die Bajonettaufnahme 156 drückt und so ein unbeabsichtigtes lösen des Bajonettverschlusses erschwert. Zum Entfernen des Werkzeuges 112 muss man dementsprechend die Bajonettstege 154 entlang des schräg angeordneten Arretierkanales 160 bewegen und dabei den Innenkorpus 116 zur Seite schieben, bevor der Bajonettverschluss endgültig gelöst werden kann.

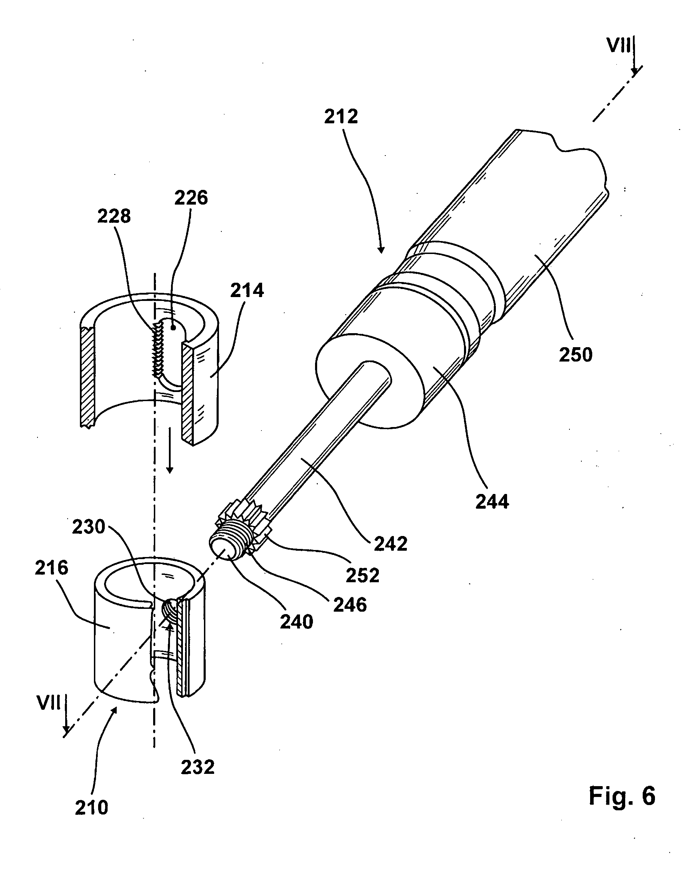

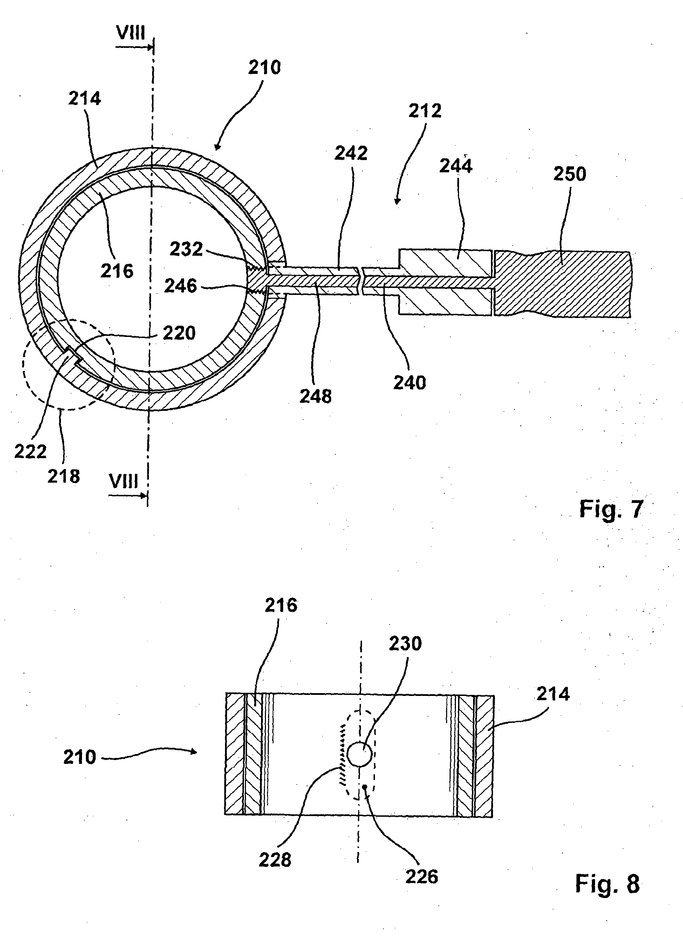

In den Fig. 6 bis 8 ist eine dritte Ausführungsform dargestellt, die sich von der ersten Ausführungsform lediglich dadurch unterscheidet, dass der Drehverschluss am Innenkorpus 216 anstatt am Außenkorpus 114 angebracht ist und dass das Werkzeug dementsprechend ausgebildet ist.

Das Wirbelsäulenimplantat gemäß dieser dritten Ausführungsform umfasst einen zylindrisch ausgebildeten Außenkorpus 214 und einen axial verschieblich darin gehaltenen ebenfalls zylindrischen Innenkorpus 216, wobei ein Führungsmittel 218 ausgebildet ist, dass die axiale Bewegung der beiden Zylinder führt und ein Verdrehen der Zylinder gegeneinander sperrt. Das Führungsmittel 218 umfasst eine an einer Außenseite des Innenkorpus 216 eingelassene, koaxial ausgerichtete Nut 220 und einen korrespondierend zur Nut 220 an einer Innenseite des Außenkorpus 214 ausgebildeten Vorsprung 222.

Im Außenkorpus 214 ist eine als Langloch ausgebildete Hebelaufnahme 226 ausgebildet, an dessen rechter oder linker vertikalen Flanke eine Anzahl von Wandungszähnen 228 ausgebildet sind. Dabei sind die Wandungszähne linear angeordnet. Im Innenkorpus 216 ist eine Transportaufnahme 230 vorgesehen, in der ein Innengewinde 232 eingelassen ist.

Die Nut 220, der Vorsprung 222, der Innenkorpus 216 und der Außenkorpus 214 sind so angeordnet, dass die Hebelaufnahme 226 mit der Transportaufnahme 230 fluchtet, so dass ein Werkzeug 212 durch die Hebelaufnahme 226 hindurch bis in die Transportaufnahme 230 gesteckt werden kann.

Das Werkzeug 212 setzt sich zusammen aus einer Instrumentenaufnahme mit einem Transportstab 240 und einem Hebeldreher 242, sowie einem Griff. Der Griff ist zweiteilig ausgebildet und setzt sich aus einem Transportgriff 244 und einem Hebelgriff 250 zusammen.

Der Transportstab 240 umfasst einen länglichen Schaft 248, an dessen proximalem Ende ein Transportgriff 244 und an dessen distalem Ende ein Außengewinde 246 angebracht ist. Der Hebeldreher 242 umfasst einen am proximalen Ende ausgebildeten Hebelgriff 250 und am distalen Ende eine Anzahl Umfangszähne 252. Der Hebeldreher 242 ist innen hohl ausgeführt und nimmt in diesem Hohlraum den Schaft 248 des Transportstabes 240 auf. Dabei ist der Schaft 248 so gewählt, dass das am distalen Ende des Schaftes 248 angeordnete Außengewinde 246 einerseits und der Hebelgriff 250 andererseits den Hebeldreher 242 mit seinen radial abstehenden Umfangszähnen 252 passgenau am Transportstab 240 halten. Durch diesen zweiteiligen Aufbau des Werkzeuges 212 ist es möglich, den Transportstab 240 unabhängig vom Hebeldreher 242 zu bewegen.

In der in den Fig. 6 bis 8 dargestellten dritten Ausführungsform sind die radial abstehenden Umfangszähne 252 über den gesamten Umfang des Hebeldrehers 242 verteilt, so dass ein kompletter Kranz von Umfangszähnen 252 entsteht. Dabei kann das Werkzeug 212 mittels eines Drehverschlusses am Implantat gehalten werden. Dieser Drehverschluss umfasst das am Transportstab 240 des Werkzeuges 212 angebrachte Außengewinde 246, sowie das in der Transportaufnahme 230 des Innenkorpus 216 angeordnete Innengewinde 232, wobei das Außengewinde 246 und das Innengewinde 232 aufeinander abgestimmt sind.

Nachfolgend wird kurz beschrieben, wie das Werkzeug 212 am Wirbelsäulenimplantat 210 verwendet werden kann:

Während eines chirurgischen Eingriffes bestimmt der behandelnde Arzt die Größe des einzusetzenden Wirbelsäulenimplantates 210. Dann kann, zum Beispiel von einem Helfer, das Werkzeug 212 mit seinem Transportstab 240 am Wirbelsäulenimplantat 210 angebracht werden. Dies geschieht durch Einschrauben des am distalen Ende des Transportstabs 240 befindlichen Außengewindes 246 in das Innengewinde 232 am Innenkorpus 216. Hierdurch entsteht eine formschlüssige und sehr stabile Verbindung zwischen dem Werkzeug 212 und dem Wirbelsäulenimplantat 210.

Zu gegebener Zeit kann der behandelnde Arzt nun das Werkzeug 212 am Griff ergreifen und hält somit nicht nur das Werkzeug 212, sondern auch das daran angeschraubte Wirbelsäulenimplantat 210 fest in der Hand. Hierzu benötigt der behandelnde Arzt nur eine einzige Hand und kann mit der anderen Hand andere Tätigkeiten ausführen.

Das über das Werkzeug 212 ergriffene Wirbelsäulenimplantat 210 kann der behandelnde Arzt nun zu der gewünschten Stelle im Körper des Patienten bringen und dort wunschgemäß platzieren. Durch die formschlüssige Verbindung zwischen dem Werkzeug 212 und dem Wirbelsäulenimplantat 210 ist es möglich, dass der behandelnde Arzt mit einer einzigen Hand Kräfte auf das Wirbelsäulenimplantat 210 ausübt, um dies auch unter schwierigen operativen Bedingungen an der gewünschten Stelle und in der gewünschten Form zu platzieren.

Ist das Wirbelsäulenimplantat 210 einmal implantiert, so kann eine Distraktion des Wirbelsäulenimplantates 210 dadurch erfolgen, dass der behandelnde Arzt den Hebelgriff 250 des Hebeldrehers 242 ergreift und in der gewünschten Richtung verdreht. Dabei greifen die am distalen Ende des Hebeldrehers 242 angebrachten Umfangszähne 252 in die Wandungszähne 228 am Außenkorpus 214 ein und verschieben diesen axial. Ein Ausweichen des Innenkorpus 216 gegenüber dem Außenkorpus 214 wird durch das Führungsmittel 218, insbesondere durch die Nut 220 und den Vorsprung 222 verhindert, so dass eine exakte axiale Verschiebung des Außenkorpus 214 erfolgt.

Durch die Hebelwirkung des Hebeldrehers 242 kann der Außenkorpus 214 auch dann entsprechend distrahiert werden, wenn auf dem Wirbelsäulenimplantat 210 bereits eine Belastung anliegt. Ein Verkanten des Wirbelsäulenimplantates 210 wird unter anderem durch das Führungselement 218 verhindert.

Nachdem das Wirbelsäulenimplantat 210 in angemessener Weise distrahiert ist, kann dieses durch Einsetzen entsprechender Sicherungsschrauben in der gewünschten Position fixiert werden. Anschließend wird das Werkzeug 212 durch Rückdrehen des Transportstabs 240 aus dem Innenkorpus 216 des Wirbelsäulenimplantates 210 herausgedreht und kann vollständig entnommen werden.

Sollte eine Korrektur des Wirbelsäulenimplantates 210 erforderlich sein, so kann dies in analoger Weise mit dem Werkzeug 212 durchgeführt werden.

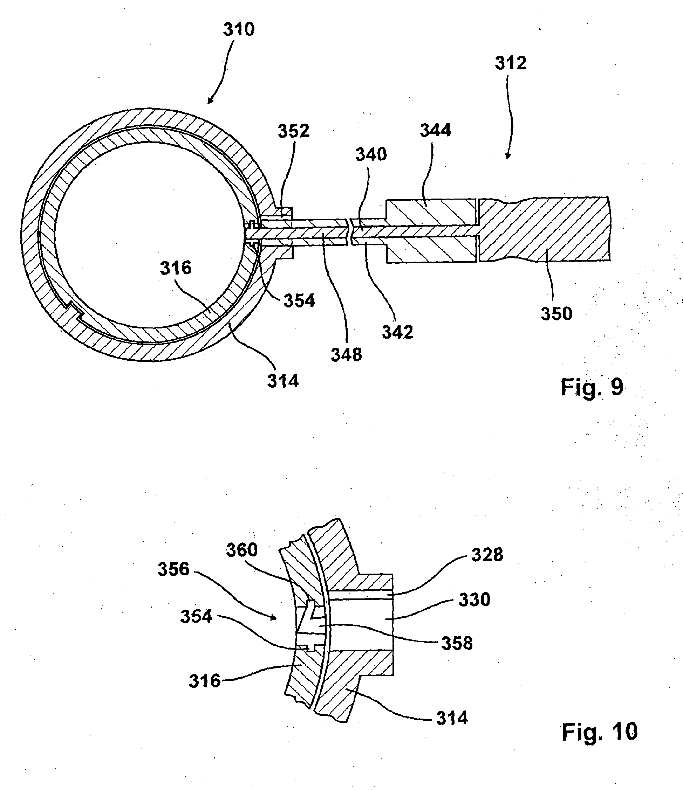

Die in den Fig. 9 und 10 dargestellte vierte Ausführungsform unterscheidet sich von der in den Fig. 6 bis 8 dargestellten dritten Ausführungsform lediglich dadurch, dass die Arretiervorrichtung nicht als Drehgewindeverschluss, sondern als Bajonettverschluss ausgeführt ist. Zu diesem Bajonettverschluss gehören zwei radial vom distalen Ende des Transportstabes 340 des Werkzeuges 312 abstehende Bajonettstege 354 und entsprechende, in der Transportaufnahme 330 des Innenkorpus 316 des Wirbelsäulenimplan-tates 310 eingelassene Bajonettaufnahmen 356. Zur Anbringung des Werkzeuges 312 am Wirbelsäulenimplantat 310 wird das Werkzeug 312 mit einer Hand am Griff ergriffen und das freie Ende des Transportstabes 340 wird mit den Bajonettstegen 354 in die Bajonettaufnahme 356 in der Transportaufnahme 330 des Innenkorpus 316 eingeführt. Anschließend wird der Transportstab 340 verdreht, so dass die Bajonettstege 354 tief in die Bajonettaufnahmen 356 eingeführt werden.

Wie insbesondere Fig. 10 zu entnehmen ist, umfasst die im Innenkorpus 316 ausgebildete Bajonettaufnahme 356 einen trichterförmig zulaufenden Eingangskanal 358, um das Einfädeln der Bajonettstege 354 zu erleichtern und einen Arretierkanal 360, wobei der Arretierkanal 360 in einem spitzen Winkel zum Eingangskanal 358 angeordnet ist. Die Anordnung in einem spitzen Winkel von vorzugsweise 80° hat den Vorteil, dass der auf den Transportstab 340 drückende Außenkorpus 314 die Bajonettstege 354 in die Bajonettaufnahme 356 drückt und so ein unbeabsichtigtes lösen des Bajonettverschlusses erschwert. Zum Entfernen des Werkzeuges 312 muss man dementsprechend die Bajonettstege 354 entlang des schräg angeordneten Arretierkanales 360 bewegen und dabei den Außenkorpus 316 zur Seite schieben, bevor der Bajonettverschluss endgültig gelöst werden kann.

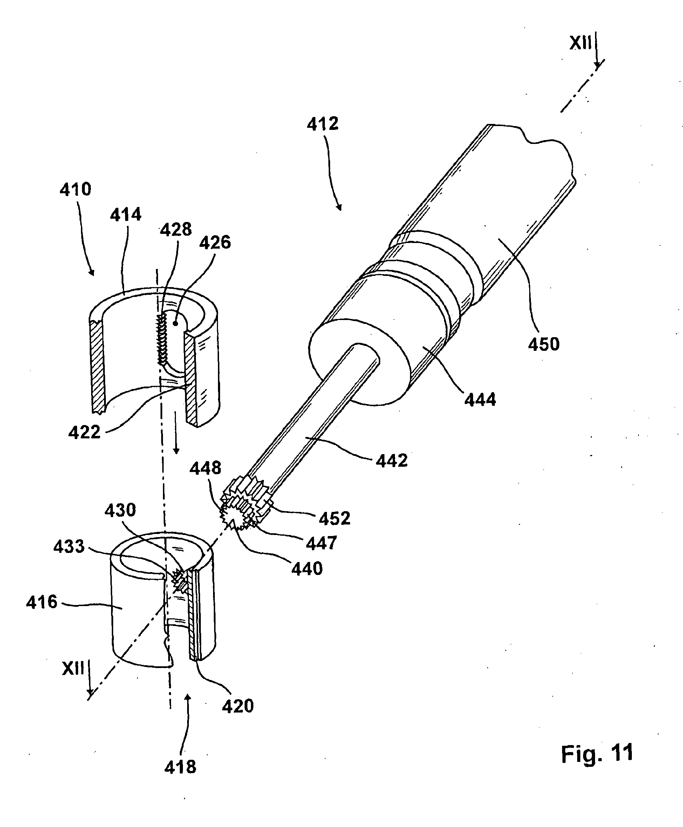

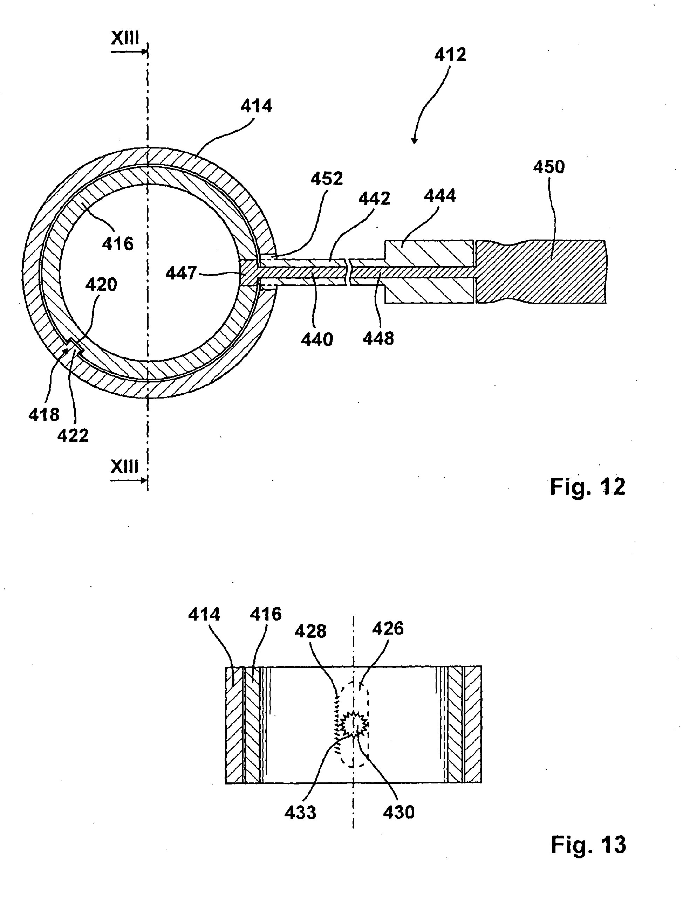

In den Fig. 11 bis 13 ist eine fünfte Ausführungsform eines erfindungsgemäßen Wirbelsäulenimplantates 410 und eines erfindungsgemäßen Werkzeuges 412 dargestellt. Das Wirbelsäulenimplantat umfasst einen Außenkorpus 414 und einen axial verschieblich darin gehaltenen Innenkorpus 416, wobei Führungsmittel 418 ausgebildet sind, die die axiale Bewegung der beiden Korpi 414, 416 führt und ein Verdrehen der Korpi gegeneinander sperrt. Das Führungsmittel 418 umfasst eine an einer Außenseite des Innenkorpus 416 eingelassene, koaxial ausgerichtete Nut 420 und einen korrespondierend zur Nut 420 an einer Innenseite des Außenkorpus 414 ausgebildeten Vorsprung 422.

Im Außenkorpus 414 ist eine als Langloch ausgeführte Hebelaufnahme 426 ausgebildet, an dessen rechter oder linker vertikalen Flanke eine Anzahl von Wandungszähnen 428 ausgebildet sind. Im Innenkorpus 416 ist eine Transportaufnahme 430 vorgesehen, in der ein Innengewinde 432 eingelassen ist.

Die Nut 420, der Vorsprung 422, der Innenkorpus 416 und der Außenkorpus 414 sind so angeordnet, dass die Hebelaufnahme 426 mit der Transportaufnahme 430 fluchtet, so dass ein Werkzeug 412 durch die Hebelaufnahme 426 hindurch bis in die Transportaufnahme 430 gesteckt werden kann.

Das Werkzeug 412 setzt sich zusammen aus einer Instrumentenaufnahme mit einem Transportstab 440 und einem Hebeldreher 442, sowie einem Griff. Der Griff ist zweiteilig ausgebildet und setzt sich aus einem Transportgriff 444 und einem Hebelgriff 450 zusammen.

Der Transportstab 440 umfasst einen länglichen Schaft 448, an dessen proximalem Ende ein Transportgriff 444 und an dessen distalem Ende ein Außenzahnkranz 447 angebracht ist. Der Hebeldreher 442 umfasst einen am proximalen Ende ausgebildeten Hebelgriff 450 und am distalen Ende eine Anzahl Umfangszähne 452. Der Hebeldreher 442 ist innen hohl ausgeführt und nimmt in diesem Hohlraum den Schaft 448 des Transportstabes 440 auf. Dabei ist der Schaft 448 so gewählt, dass der am distalen Ende des Schaftes 448 angeordnete Außenzahnkranz 446 einerseits und der Hebelgriff 450 andererseits den Hebeldreher 442 mit seinen radial abstehenden Umfangszähnen 452 passgenau am Transportstab 440 halten. Durch diesen zweiteiligen Aufbau des Werkzeuges 412 ist es möglich, den Transportstab 440 unabhängig vom Hebeldreher 442 zu bewegen.

In der in den Fig. 11 bis 13 dargestellten fünften Ausführungsform sind die radial abstehenden Umfangszähne 452 über den gesamten Umfang des Hebeldrehers 442 verteilt, so dass ein kompletter Kranz von Umfangszähnen 452 entsteht. Dabei kann das Werkzeug 412 mittels eines Zahnverschlusses am Implantat gehalten werden. Dieser Zahnverschluss umfasst den am Transportstab 440 des Werkzeuges 412 angebrachten Außenzahnkranz 447, sowie den in der Transportaufnahme 430 des Innenkorpus 416 angeordneten Innenzahnkranz 432, wobei der Außenzahnkranz 446 und der Innenzahnkranz 432 aufeinander abgestimmt sind.

Nachfolgend wird kurz beschrieben, wie das Werkzeug 412 am Wirbelsäulenimplantat 410 verwendet werden kann:

Während eines chirurgischen Eingriffes bestimmt der behandelnde Arzt die Größe des einzusetzenden Wirbelsäulenimplantates 410. Dann kann, zum Beispiel von einem Helfer, das Werkzeug 412 mit seinem Arretierstab 440 am Wirbelsäulenimplantat 410 angebracht werden. Dies geschieht durch Einschieben des am distalen Ende des Transportstabs 440 befindlichen Außenzahnkranzes 247 in den Innenzahnkranz 432 am Innenkorpus 416. Hierdurch entsteht eine formschlüssige und sehr stabile Verbindung zwischen dem Werkzeug 412 und dem Wirbelsäulenimplantat 410.

Zu gegebener Zeit kann der behandelnde Arzt nun das Werkzeug 412 am Griff 444 und/oder am Griff 450 ergreifen und hält somit nicht nur das Werkzeug 412, sondern auch das daran angeschraubte Wirbelsäulenimplantat 410 fest in der Hand. Hierzu benötigt der behandelnde Arzt nur eine einzige Hand und kann mit der anderen Hand andere Tätigkeiten ausführen.

Das über das Werkzeug 412 ergriffene Wirbelsäulenimplantat 410 kann der behandelnde Arzt nun zu der gewünschten Stelle im Körper des Patienten bringen und dort wunschgemäß platzieren. Durch die formschlüssige Verbindung zwischen dem Werkzeug 412 und dem Wirbelsäulenimplantat 410 ist es möglich, dass der behandelnde Arzt mit einer einzigen Hand Kräfte auf das Wirbelsäulenimplantat 410 ausübt, um dies auch unter schwierigen operativen Bedingungen an der gewünschten Stelle und in der gewünschten Form zu platzieren.

Ist das Wirbelsäulenimplantat 410 einmal implantiert, so kann eine Distraktion des Wirbelsäulenimplantates 410 dadurch erfolgen, dass der behandelnde Arzt den Hebelgriff 450 des Hebeldrehers 442 ergreift und in der gewünschten Richtung verdreht. Dabei greifen die am distalen Ende des Hebels 442 angebrachten Umfangszähne 452 in die Wandungszähne 428 am Außenkorpus 414 ein und verschieben diesen axial. Ein Ausweichen des Innenkorpus 416 gegenüber dem Außenkorpus 414 wird durch das Führungsmittel 418, insbesondere durch die Nut 420 und den Vorsprung 422 verhindert, so dass eine exakte axiale Verschiebung des Außenkorpus. 414 erfolgt.

Durch die Hebelwirkung des Hebeldrehers 442 kann der Außenkorpus 414 auch dann entsprechend distrahiert werden, wenn auf dem Wirbelsäulenimplantat 410 bereits eine Belastung anliegt. Ein Verkanten des Wirbelsäulenimplantates 410 wird unter anderem durch das Führungsmittel 418 verhindert.

Nachdem das Wirbelsäulenimplantat 410 in angemessener Weise distrahiert ist, kann dieses durch Einsetzen entsprechender Sicherungsschrauben in der gewünschten Position fixiert werden. Anschließend wird das Werkzeug 412 durch Rückdrehen des Transportstabs 440 aus dem Innenkorpus 416 des Wirbelsäulenimplantates 410 herausgedreht und kann vollständig entnommen werden.

Sollte eine Korrektur des Wirbelsäulenimplantates 410 erforderlich sein, so kann dies in analoger Weise mit dem Werkzeug 412 durchgeführt werden.

## Patentansprüche

1. Wirbelsäulenimplantat mit einem Außenkorpus (14, 114, 214, 314, 414) und mit einem axial verschieblich darin gehaltenen Innenkorpus (16, 116, 216, 316, 416),
wobei entweder am Außenkorpus (14, 114, 216, 316, 416) eine Transportaufnahme (30, 130, 230, 330, 430) und am Innenkorpus (16, 116, 216, 316, 416) eine Hebelaufnahme (26, 226, 426) vorgesehen ist, oder wobei am Außenkorpus (14, 114, 214, 314, 414) eine Hebelaufnahme (26, 226, 426) und am Innenkorpus (16, 116, 216, 316, 416) eine Transportaufnahme (30, 130, 230, 330, 430) vorgesehen ist, wobei die Hebel- (26, 226, 426) und die Transportaufnahme (30, 130, 230, 330, 430) derart miteinander korrespondierend ausgebildet sind, dass darin ein von außen in das Wirbelsäulenimplantat (10, 110, 210, 310, 410) einführbares, das Wirbelsäulenimplantat (10, 110, 210, 310, 410) tragende und das Wirbelsäulenimplantat (10, 110, 210, 310, 410) distrahierende Werkzeug (12, 112, 212, 312, 412) aufnehmbar ist, wobei in der Transportaufnahme (30, 130, 230, 330, 430) Mittel zum Arretieren des Werkzeuges (12, 112, 212, 312, 412) ausgebildet sind, und wobei in der Hebelaufnahme (26, 226, 426) Mittel zum Eingriff eines Hebel drehers (42, 242, 342, 442) des Werkzeuges (12, 112, 212, 312, 412) vorgesehen sind, so dass das Wirbelsäulenimplantat (10, 110, 210, 310, 410) distrahiert werden kann,
**dadurch gekennzeichnet,**
**dass** am Innen- (16, 216, 416) und am Außenkorpus (14, 214, 414) Führungsmittel (18, 218, 418) vorgesehen sind, die ein axiales Verschieben des Innenkorpus (16, 216, 416) gegenüber dem Außenkorpus (14, 214, 414) erlauben, die aber ein Verdrehen des Innenkorpus (16, 216, 416) gegenüber dem Außenkorpus (14, 214, 414) verhindern, und dass die Führungsmittel (18, 218, 418) nach dem Nut-Feder-Prinzip ausgebildet sind.

2. Wirbelsäulenimplantat nach Anspruch 1,
**dadurch gekennzeichnet,**
**dass** die Mittel zum Arretieren des Werkzeuges (10, 210) ein in der Transportaufnahme (30, 230) ausgebildetes Innengewinde (32, 232) umfassen.

3. Wirbelsäulenimplantat nach Anspruche 1,
**dadurch gekennzeichnet,**
**dass** die Mittel zum Arretieren des Werkzeuges (112, 312) eine in der Transportaufnahme (130, 330) ausgebildete Bajonettaufnahme (156, 356) umfassen.

4. Wirbelsäulenimplantat nach Anspruch 1,
**dadurch gekennzeichnet,**
**dass** die Mittel zum Arretieren des Werkzeuges (412) einen in der Transportaufnahme (430) ausgebildeten Innenzahnkranz (456) umfassen.

5. Wirbelsäulenimplantat nach einem der vorangehenden Ansprüche, **dadurch gekennzeichnet,**
**dass** die Mittel zum Eingriff eines Hebeldrehers (42, 242, 342, 442) des Werkzeuges (12, 112, 212, 312, 412) eine als Langloch ausgebildete Hebelaufnahme (26, 226, 426) umfassen, wobei an einer Längsseite der Hebelaufnahme (26, 226, 426) eine Anzahl in die Hebelaufnahme (26, 226, 426) hineinragender Wandungszähne (28, 228, 328, 428) ausgebildet sind.

6. Wirbelsäulenimplantat nach Anspruch 5,
**dadurch gekennzeichnet,**
**dass** die Wandungszähne (28, 228, 328, 428) linear angeordnet sind.

7. Werkzeug für ein insbesondere nach einem der Ansprüche 1 bis 6 ausgebildetes Wirbelsäulenimplantat, mit einer stabähnlichen Instrumentenaufnahme und einem daran angebrachten Griff,
wobei die Instrumentenaufnahme sowohl Mittel zum Transportieren des Wirbelsäulenimplantates (10, 110, 210, 310, 410), als auch Mittel zum Distrahieren des Wirbelsäulenimplantates (10, 110, 210, 310, 410) aufweist, und dass der Griff zweigeteilt ausgeführt ist und einen Transportgriff (44, 144, 244, 344, 444) und einen Hebelgriff (50, 150, 250, 350, 450) umfasst, so dass die Mittel zum Transportieren und die Mittel zum Distrahieren separat bedient werden können, dass die Mittel zum Transportieren des Wirbelsäulenimplantates (10, 110) einen länglichen, hohl ausgeführten Transportstab (40, 140) aufweisen, an dessen proximalen Ende ein Transportgriff (44, 144) ausgebildet ist und an dessen distalem Ende Mittel zum Arretieren des Werkzeuges (12, 112) am Wirbelsäulenimplantat (10, 110) vorgesehen sind, dass die Mittel zum Distrahieren einen einen länglichen Schaft (48) aufweisenden Hebeldreher (42) aufweisen, an dessen proximalen Ende ein Hebelgriff (50, 150) angebracht ist und an dessen distalem Ende über den Umfang verteilt eine Anzahl von Umfangszähnen (52) ausgebildet sind, und dass der Schaft (48) des Hebeldrehers (42) im Inneren des hohl ausgeführten Transportstabs (40, 140) angeordnet ist.

8. Werkzeug nach Anspruch 7,
**dadurch gekennzeichnet,**
**dass** die Mittel zum Arretieren des Werkzeuges (12, 112, 212, 312, 412) am Wirbelsäulenimplantat (10, 110, 210, 310, 410) ein am distalen Ende des Transportstabes (40, 240) angebrachtes Außengewinde (46, 246) oder am distalen Ende des Transportstabes (140, 340) angebrachte Bajonettstege (154, 354) oder einen am distalen Ende des Transportstabes (440) angebrachten Außenzahnkranz (447) umfassen.

9. Werkzeug nach einem der Ansprüche 7 bis 8,
**dadurch gekennzeichnet,**
**dass** die Mittel zum Arretieren des Werkzeuges (12, 112, z2, 312, 412) am Wirbelsäulenimplantat (10, 110, 210, 310, 410) und die Umfangszähne (52, 252, 452) derart angeordnet sind, dass die Umfangszähne (52, 252, 452) in die Wandungszähne (28, 228, 328, 428) der Hebelaufnahme (26, 226, 426) des Wirbelsäulenimplantates (10, 110, 210, 310, 410) eingreifen, während die Mittel zum Arretieren an der Transportaufnahme (14, 114, 214, 314, 414) des Wirbelsäulenimplantates (10, 110, 210, 310, 410) im Eingriff sind.

## Claims

1. A spinal implant with an outer corpus (14, 114, 214, 314, 414) and with an inner corpus (16, 116, 216, 316, 416) held therein in an axially displaceable manner,
wherein either a transport admission (30, 130, 230, 330, 430) is provided on the outer corpus (14, 114, 214, 314, 414) and a lever admission (26, 226, 426) is provided on the inner corpus (16, 116, 216, 316, 416) or wherein a lever admission (26, 226, 426) is provided on the outer corpus (14, 114, 214, 314, 414) and a transport admission (30, 130, 230, 330, 430) is provided on the inner corpus (16, 116, 216, 316, 416), wherein the lever admission (26, 226, 426) and the transport admission (30, 130, 230, 330, 430) are configured to correspond to each other in such a manner that a tool (12, 112, 212, 312, 412), insertable into the spinal implant (10, 110, 210, 310, 410) from the outside, supporting the spinal implant (10, 110, 210, 310, 410) and distracting the spinal implant (10, 110, 210, 310, 410), is receivable therein, wherein means for locking the tool (12, 112, 212, 312, 412) are provided in the transport admission (30, 130, 230, 330, 430) and wherein means for engagement of a lever rotator (42, 242, 342, 442) of the tool (12, 112, 212, 312, 412) are provided in the lever admission (26, 226, 426), so that the spinal implant (10, 110, 210, 310, 410) can be distracted,
**characterized in that**
guiding means (18, 218, 418), which allow for an axial displacement of the inner corpus (16, 216, 416) relative to the outer corpus (14, 214, 414) but which prevent a rotation of the inner corpus (16, 216, 416) relative to the outer corpus (14, 214, 414), are provided on the inner corpus (16, 216, 416) and on the outer corpus (14, 214, 414) and that the guiding means (18, 218, 418) are configured according to the tongue and groove principle.

2. The spinal implant according to claim 1,
**characterized in that**
the means for locking the tool (10, 210) comprise an inner thread (32, 332) arranged in the transport admission (30, 230).

3. The spinal implant according to claim 1,
**characterized in that**
the means for locking the tool (112, 312) comprise a bayonet admission (156, 356) arranged in the transport admission (130, 330).

4. The spinal implant according to claim 1,
**characterized in that**
the means for locking the tool (412) comprise an inner gear ring (456) arranged in the transport admission (430).

5. The spinal implant according to one of the afore-mentioned claims, **characterized in that**
the means for engaging a lever rotator (42, 242, 342, 442) of the tool (12, 112, 212, 312, 412) comprise a lever admission (26, 226, 426) configured as a long hole, wherein a plurality of wall teeth (28, 228, 328, 428) are formed on a longitudinal side of the lever admission (26, 226, 426) and protrude into the lever admission (26, 226, 426).

6. The spinal implant according to claim 5,
**characterized in that**
the wall teeth (28, 228, 328, 428) are disposed linearly.

7. A tool for a spinal implant more specifically designed according to one of the claims 1 to 6, with a rod-type instrument admission and a handle attached to it, wherein the instrument admission comprises means for transporting the spinal implant (10, 110, 210, 310, 410) as well as means to distract the spinal implant (10, 110,210, 310, 410) and that the handle is designed in two parts and comprises a transport handle (44, 144, 244, 344, 444) and a lever handle (50, 150, 250, 350, 450), so that the means for transport and the means for distraction can be operated separately, that the means for transport of the spinal implant (10, 110) have an oblong hollow transport rod (40, 140) at the proximal end of which a transport handle (44,144) is formed and at the distal end of which means for locking the tool (12, 112) to the spinal implant (10, 110) are provided, that the means for distraction have a lever rotator (42) having an oblong shaft (48), at the proximal end of which a lever handle (50, 150) is disposed and at the distal end of which a plurality of circumferential teeth (52) are distributed along its circumference, and that the shaft (48) of the lever rotator (42) is disposed inside the hollow transport rod (40, 140).

8. The tool according to claim 7,
**characterized in that**
the means for locking the tool (12, 112, 212, 312, 412) on the spinal implant (10, 110, 210, 310, 410) comprise an outer thread (46, 246) attached to the distal end of the transport rod (40, 240) or bayonet bars (154, 354) attached to the distal end of the transport rod (140, 340) or an outer gear ring (447) attached to the distal end of the transport rod (440).

9. The tool according to one of the claims 7 to 8,
**characterized in that**
the means for locking the tool (12, 112, 212, 312, 412) on the spinal implant (10, 110, 210, 310, 410) and the circumferential teeth (52, 252, 452) are disposed so that the circumferential teeth (52, 252, 452) engage with the wall teeth (28, 228, 328, 428) of the lever admission (26, 226, 426) of the spinal implant (10, 110, 210, 310, 410), while the means for locking are engaged with the transport admission (14, 114, 214, 314, 414) of the spinal implant (10, 110, 210, 310, 410).

## Revendications

1. Implant pour colonne vertébrale avec un corps extérieur (14, 114, 214, 314, 414) et un corps intérieur (16, 116, 216, 316, 416) maintenu dans celui-ci de manière axialement déplaçable,
où soit un logement de transport (30, 130, 230, 330, 430) est prévu au niveau du corps extérieur (14, 114, 214, 314, 414) et un logement de levier (26, 226, 426) est prévu au niveau du corps intérieur (16, 116, 216, 316, 416), ou bien où un logement de levier (26, 226, 426) est prévu au niveau du corps extérieur (14, 114, 214, 314, 414) et un logement de transport (30, 130, 230, 330, 430) est prévu au niveau du corps intérieur (16, 116, 216, 316, 416), où le logement de levier (26, 226, 426) et le logement de transport (30, 130, 230, 330, 430) sont formés de manière à correspondre l'un avec l'autre de telle façon qu'un outil (12, 112, 212, 312, 412), insérable depuis l'extérieur dans l'implant de colonne vertébrale (10, 110, 210, 310, 410), portant l'implant de colonne vertébrale (10, 110, 210, 310, 410) et distractant l'implant de colonne vertébrale (10, 110, 210, 310, 410) y est recevable, où des moyens de blocage de l'outil (12, 112, 212, 312, 412) sont formés dans le logement de transport (30, 130, 230, 330, 430) et où des moyens d'engagement d'un rotateur de levier (42, 242, 342, 442) de l'outil (12, 112, 212, 312, 412) sont prévus dans le logement de levier (26, 226, 426), de manière à pouvoir distraire l'implant de colonne vertébrale (10, 110, 210, 310, 410), **caractérisé en ce que**
sont prévus sur le corps intérieur (16, 216, 416) et sur le corps extérieur (14, 214, 414) des moyens de guidage (18, 218, 418) qui permettent un déplacement axial du corps intérieur (16, 216, 416) par rapport au cours extérieur (14, 214, 414) mais qui empêchent une rotation du corps intérieur (16, 216, 416) par rapport au corps extérieur (14, 214, 414) et que les moyens de guidage (18, 218, 418) sont formés sur le principe d'un assemblage à rainure et languette.

2. Implant de colonne vertébrale selon la revendication 1, **caractérisé en ce que**
les moyens de blocage de l'outil (10, 210) comprennent un taraudage (32, 232) formé dans le logement de transport (30, 230).

3. Implant de colonne vertébrale selon la revendication 1, **caractérisé en ce que**
les moyens de blocage de l'outil (112, 312) comprennent un logement de baïonnette (156, 356) formé dans le logement de transport (130, 330).

4. Implant de colonne vertébrale selon la revendication 1, **caractérisé en ce que**
les moyens de blocage de l'outil (412) comprennent une couronne dentée intérieure (456) formée dans le logement de transport (430).

5. Implant de colonne vertébrale selon l'une quelconque des revendications précédentes,
**caractérisé en ce que**
les moyens d'engagement d'un rotateur de levier (42, 242, 342, 442) de l'outil (12, 112, 212, 312, 412) comprennent un logement de levier (26, 226, 426) configuré en forme de trou oblong, où une pluralité de dents de paroi (28, 228, 328, 428) faisant saillie dans le logement de levier (26, 226, 426) est formée sur un côté longitudinal du logement de levier (26, 226, 426).

6. Implant de colonne vertébrale selon la revendication 5, **caractérisé en ce que**
les dents de paroi (28, 228, 328, 428) sont disposées de manière linéaire.

7. Outil pour un implant de colonne vertébrale configuré en particulier selon l'une des revendications 1 à 6 avec un logement d'instrument en forme de tige et une poignée fixée sur celui-ci, où le logement d'instrument comporte à la fois des moyens de transport de l'implant de colonne vertébrale (10, 110, 210, 310, 410) et des moyens de distraction de l'implant de colonne vertébrale (10, 110, 210, 310, 410) et que la poignée est configurée en deux parties et comprend une poignée de transport (44, 144, 244, 344, 444) et une poignée de levier (50, 150, 250, 350, 450), de sorte que les moyens de transport et les moyens de distraction peuvent être maniés séparément, que les moyens de transport de l'implant de colonne vertébrale (10, 110) comportent une tige de transport (40,140) oblongue et creuse, à l'extrémité proximale de laquelle est formé une poignée de transport (44, 144) et à l'extrémité distale de laquelle sont prévus des moyens de blocage de l'outil (12, 112) sur l'implant de colonne vertébrale (10, 110), que les moyens de distraction comportent un rotateur de levier (42) comportant un manche (48) oblong, à l'extrémité proximale duquel est fixé une poignée de levier (50, 150) et à l'extrémité distale duquel sont formés une pluralité de dents circonférentielles (52) distribuées autour de sa circonférence et que le manche (48) du rotateur de levier (42) est disposé à l'intérieur de la tige de transport (40, 140) creuse.

8. Outil selon la revendication 7,
**caractérisé en ce que**
les moyens de blocage de l'outil (12, 112, 212, 312, 412) au niveau de l'implant de colonne vertébrale (10, 110, 210, 310, 410) comprennent un filet (46, 246) fixé à l'extrémité distale de la tige de transport (40, 240) ou des tiges de baïonnette (154, 354) fixées à l'extrémité distale de la tige de transport (140, 340) ou une couronne dentée extérieure (447) fixée à l'extrémité distale de la tige de transport (440).

9. Outil selon l'une des revendications 7 à 8,
**caractérisé en ce que**
les moyens de blocage de l'outil (12, 112, 212, 312, 412) au niveau de l'implant de colonne vertébrale (10, 110, 210, 310, 410) et les dents circonférentielles (52, 252, 452) sont disposés de telle manière que les dents circonférentielles (52, 252, 452) s'engagent dans les dents de paroi (28, 228, 328, 428) du logement de levier (26, 226, 426) de l'implant de colonne vertébrale (10, 110, 210, 310, 410), tandis que les moyens de blocage sont engagés dans le logement de transport (14, 114, 214, 314, 414) de l'implant de colonne vertébrale (10, 110, 210, 310, 410).
